# FASCICULE DE BREVET EUROPEEN

(11) **EP 0 440 547 B1**
(45) Date de publication et mention de la délivrance du brevet: **13.07.1994**
(21) Numéro de dépôt: 91400198.7
(22) Date de dépôt: 29.01.1991
(51) Int. Cl.: A61K 7/09

(54) **Composition cosmétique réductrice pour la déformation permanente des cheveux à base d'un ester de l'acide thioglycolique et de N-acyl(C2-C4)cystéamine et son procédé de mise en oeuvre**
Reduzierende kosmetische Zusammensetzung zur Haardauerwellung auf Basis eines Esters der Thioglycolsäure und eines N-Acyl(C2-C4)-Cysteamines sowie Verfahren zu deren Anwendung
Cosmetic reducing composition for permanent hair perming based on a thioglycolic acid ester and a N-acyl(C2-C4)-cysteamine and its method of application

(30) Priorité: 29.01.1990 FR 9000993
(43) Date de publication de la demande: 07.08.1991
(73) Titulaire: L'OREAL, 75008 Paris (FR)
(72) Inventeur: de Labbey, Arnaud, F-93600 Aulnay-Sous-Bois (FR)
(74) Mandataire: Stalla-Bourdillon, Bernard

(56) Documents cités:
- EP-A- 0 261 387
- PATENT ABSTRACTS OF JAPAN, vol. 12, no. 405 (C-539)[3252], 26 octobre 1988; & JP-A-63146808 (Kao Corp.) 18-06-1988
- PATENT ABSTRACTS OF JAPAN, vol. 6, no. 139 (C-116)[1017], 28 juillet 1982;& JP-A-57 62 217 (KAO SEKKEN K.K.) 15-04-1982

## Description

La présente invention a pour objet une composition cosmétique réductrice pour la déformation permanente des cheveux, à base d'un ester d'acide thioglycolique et d'une N-acyl(C₂-C₄)cystéamine ainsi qu'un procédé de déformation permanente des cheveux.

La technique classique pour réaliser une déformation permanente des cheveux consiste à réaliser, dans un premier temps, l'ouverture des liaisons disulfures de la kératine à l'aide d'une composition contenant un agent réducteur (étape de réduction), puis, après avoir de préférence rincé la chevelure, à reconstituer dans un second temps lesdites liaisons disulfures en appliquant, sur les cheveux sous tension, une composition oxydante (étape d'oxydation, dite aussi de fixation) de façon à donner aux cheveux la forme définitive désirée. Cette technique permet indifféremment de réaliser soit une ondulation des cheveux soit un défrisage ou décrêpage.

Parmi les agents réducteurs connus permettant de réaliser le premier temps d'une opération de permanente, on utilise généralement des mercaptans tels que l'acide thioglycolique, l'acide thiolactique ou un mélange de ces acides ainsi que leurs esters par exemple le monothioglycolate de glycérol ou de glycol.

Il a également été proposé, dans la demande de brevet japonais n° 82-62.217, en tant qu'agent réducteur, l'association de cystéamine ou l'un de ses dérivés N-alkyle et de certains agents réducteurs classiques tels que l'acide thioglycolique, la cystéine, le thioglycérol et l'acide thiolactique ceci en vue de pallier, dans la mesure du possible, au problème posé par l'odeur désagréable des agents réducteurs classiques.

Dans ce même esprit, il a également été proposé, dans la demande de brevet européen n° 0.261.387, une composition réductrice contenant l'association d'un ester de l'acide thioglycolique et de cystéamine ou de l'un de ses sels.

On connait également les associations d'agents réducteurs telles que l'association d'une N-acylcystéamine avec un autre agent réducteur classique décrite dans la demande de brevet japonais n° 63-146.808.

On a toutefois constaté que ces compositions réductrices contenant de la cystéamine présentaient cependant l'inconvénient de dégager une odeur incommodante non pas tellement lors de l'application mais au cours du temps au niveau des cheveux à l'état humide et ceci même après des lavages répétés, cette odeur étant d'autant plus accentuée chez les personnes ayant une transpiration importante du cuir chevelu.

On vient maintenant de constater que l'on pouvait remédier à cet inconvénient, de façon tout à fait satisfaisante, en utilisant une N-acyl(C₂-C₄)cystéamine dans les compositions réductrices à base d'un ester d'acide thioglycolique.

La présente invention a donc pour objet une composition cosmétique réductrice pour la déformation permanente des cheveux, contenant en tant qu'agents réducteurs un ester de l'acide thioglycolique et une N-acyl (C₂-C₄)cystéamine, le pH de ladite composition étant inférieur à 8.

Les études qui ont été réalisées, ont par ailleurs permis de montrer que par l'emploi de la composition réductrice selon l'invention l'état des cheveux traités étaient beaucoup plus satisfaisants sur le plan cosmétique et que par ailleurs la composition pouvait présenter avantageusement une plus grande innocuité par rapport aux compositions de l'art antérieur.

Selon un mode de réalisation préféré, l'ester de l'acide thioglycolique est le monothioglycolate de glycérol ou le monothioglycolate d'éthylène glycol.

Par ailleurs la N-acyl(C₂-C₄)cystéamine est de préférence la N-acétylcystéamine.

Dans la composition réductrice selon l'invention, l'ester de l'acide thioglycolique est généralement présent à une concentration comprise entre 5 et 30% en poids par rapport au poids total de la composition et la concentration en N-acyl(C₂C₄)cystéamine est de préférence comprise entre 0,25 et 30% en poids par rapport au poids total de la composition.

Le rapport en poids entre la N-acyl(C₂-C₄)cystéamine et l'ester de l'acide thioglycolique est compris entre 0,05 et 2.

Selon un mode de réalisation préféré, la composition réductrice contient également un agent tensio-actif de type non ionique, anionique, cationique ou amphotère et parmi ceux-ci on peut citer les alkylsulfates, les alkylbenzènesulfates, les alkyléthersulfates, les alkylsulfonates, les sels d'ammonium quaternaire, les alkylbétaines, les alkylphénols oxyéthylénés, les alcanolamides d'acides gras, les esters d'acides gras oxyéthylénés ainsi que d'autres agents tensio-actifs non ioniques du type hydroxypropyléther.

Lorsque la composition réductrice contient au moins un agent tensio-actif, celui-ci est généralement présent à une concentration maximale de 30% en poids, mais de préférence comprise entre 0,5 et 10% par rapport au poids total de la composition réductrice.

Dans le but d'améliorer les propriétés cosmétiques des cheveux ou encore d'en atténuer ou d'éviter leur dégradation, la composition réductrice peut également contenir un agent traitant de nature cationique, anionique, non ionique ou amphotère.

Parmi les agents traitants particulièrement préférés, on peut notamment citer ceux décrits dans les brevets français n° 2.598.613, et n° 2.470.596. On peut également utiliser comme agents traitants des silicones volatiles ou non, linéaires ou cycliques et leurs mélanges, les polydiméthylsiloxanes, les polyorganosiloxanes quarternisés tels que ceux décrits dans la demande de brevet français n° 2.535.730, les polyorganosiloxanes à groupements aminoalkyles modifiés par des groupements alkoxycarbonylalkyles tels que ceux décrits dans le brevet US n° 4.749.732, des polyorganosiloxanes tels que le copolymère polydiméthylsiloxane-polyoxyalkyle du type Diméthicone Copolyol, un polydiméthylsiloxane à groupements terminaux stéaroxy (stéaroxydiméthicone), un copolymère polydiméthylsiloxane-dialkylammonium acétate ou un copolymère polydiméthyl -siloxane polyalkylbétaïne décrits dans le brevet Britannique n° 2.197.352, des polysiloxanes organo modifiés par des groupements mercapto ou mercaptoalkyle tels que ceux décrits dans le brevet Français n° 1.530.369 et la demande de brevet européen n° 0.295.780, ainsi que des silanes tels que le stéaroxytriméthylsilane.

La composition réductrice peut également contenir d'autres ingrédients traitants tels que des aminoacides basiques (tels que la lysine, l'arginine) ou acides (tels que l'acide glutamique, l'acide aspartique), des peptides et leurs dérivés, des agents de gonflement et de pénétration ou permettant de renforçer l'efficacité du réducteur tels que le mélange SiO₂/PDMS, le diméthylisosorbitol, l'urée et ses dérivés, les alkyléthers d'alkylèneglycol ou de dialkylèneglycol tels que par exemple le monométhyléther de propylèneglycol, le monométhyléther de dipropylèneglycol, le monoéthyléther de l'éthylèneglycol et le monoéthyléther du diéthylèneglycol, des alcanediols en C₃-C₆ tels que par exemple le propanediol-1,2 et le butanenediol-1,2, la pyrrolidone-2, l'imidazolidinone-2 ainsi que d'autres composés tels que des alcools gras, des dérivés de la lanoline, des ingrédients actifs tels que l'acide panthothénique, des agents antichutes, des agents antipélliculaires, des épaississants, des agents de suspension, des agents séquestrants, des agents opacifiants, des colorants, des filtres solaires ainsi que des parfums et conservateurs.

La composition réductrice selon l'invention se présente essentiellement sous forme aqueuse notamment sous la forme d'une lotion épaissie ou non, d'une crème ou d'un gel. Le pH de la composition réductrice est de préférerence compris entre 5 et 7,5 et celui-ci peut être ajusté au moyen d'un régulateur de pH qui est soit un agent acidifiant choisi parmi l'acide lactique, l'acide chlorhydrique, l'acide citrique ou l'acide phosphorique soit un agent alcalinisant choisi parmi l'ammoniaque, les mono, di et triéthanolamines, les carbonates ou bicarbonates alcalins ou d'ammonium ou encore au moyen de tampons tels que par exemple le phosphate mono et dipotassique et le carbonate acide d'ammonium.

La composition réductrice selon l'invention peut également contenir un solvant tel que par exemple de l'éthanol, du propanol, ou de l'isopropanol ou encore du glycérol à une concentration maximale de 20% par rapport au poids total de la composition.

Les esters des acides thioglycoliques n'étant pas très stables en milieu aqueux, il est préférable de réaliser la composition au moment de l'emploi par mélange des différents ingrédients.

De façon à pouvoir réaliser la composition réductrice, les ingrédients se présentent donc conditionnés en plusieurs parties, l'ester de l'acide thioglycolique étant toujours en milieu anhydre.

Le conditionnement en plusieurs parties peut prendre plusieurs formes.

Selon un premier mode, une première partie contient l'ester de l'acide thioglycolique en milieu anhydre de préférence dans du glycérol et la deuxième partie contient en milieu aqueux la N-acyl(C₂-C₄)cystéamine.

Selon un deuxième mode, la première partie contient l'ester de l'acide thioglycolique et la N-acyl(C₂-C₄)cystéamine en milieu anhydre, de préférence dans du glycérol et la deuxième partie contient le milieu aqueux.

L'agent régulateur de pH est généralement présent dans la partie contenant le milieu aqueux, toutefois dans le premier mode de réalisation celui-ci peut être présent en milieu aqueux dans une troisième partie lorsqu'il peut y avoir incompatibilité avec la N-acyl(C₂-C₄)cystéamine.

Selon ces formes de conditionnement, les adjuvants cosmétiques peuvent être présents soit dans la phase aqueuse, soit dans la phase anhydre à condition bien entendu que l'ester de l'acide thioglycolique reste en milieu totalement anhydre et que les composés soient compatibles entre eux.

La présente invention a également pour objet un procédé d'ondulation des cheveux dans lequel on applique une composition réductrice telle que définie ci-dessus sur des cheveux mouillés préalablement roulés sur des rouleaux ayant de 4 à 20 mm. de diamètre, la composition pouvant éventuellement être appliquée au fur et à mesure de l'enroulage des cheveux; on laisse ensuite agir la composition réductrice pendant un temps de 5 à 60 min. de préférence de 5 à 30 min. à une température pouvant être comprise entre 20 et 55°C.

On rince ensuite abondamment et après quoi on applique sur les cheveux enroulés une composition oxydante, permettant de reformer les liaisons disulfures de la kératine, pendant un temps de pose de 2 à 10 min.. Après avoir enlevé les rouleaux, on rince abondamment la chevelure.

La composition d'oxydation ou oxydante du type couramment utilisé contient, comme agent oxydant, des peroxydes tels que l'eau oxygénée ou éventuellement le peroxyde d'urée, un bromate alcalin, un persel ou un mélange de bromate alcalin et d'un persel.

La concentration en eau oxygénée peut varier de 1 à 10 volumes, mais est de préférence de 8 volumes, la concentration en bromate alcalin est de 1 à 12% et celle en persel de 0,1 à 15% en poids par rapport au poids total de la composition oxydante.

Le pH de la composition oxydante peut varier entre 2 et 7 mais de préférence entre 4 et 6.

L'eau oxygénée peut être stabilisée par exemple par la phénacétine, l'acétalinide, les phosphates mono et trisodiques ou par le sulfate d'hydroxy-8 quinoléine.

L'oxydation peut être immédiate ou différée.

Les compositions oxydantes peuvent également contenir des agents alcalinisants ou acidifiants, des agents conservateurs, des agents sequestrants, des opacifiants et des agents traitants ou des subtances actives telles que définies ci-dessus pour la composition réductrice.

La présente invention a également pour objet un procédé de défrisage ou de décrêpage des cheveux dans lequel on applique sur les cheveux une composition réductrice selon l'invention, puis on soumet les cheveux à une déformation mécanique permettant de les fixer dans leur nouvelle forme, par une opération de lissage des cheveux avec un peigne à large dents, avec le dos d'un peigne ou à la main. Après un temps de pose de 5 à 60 min., en particulier de 5 à 30 min., on procède alors à un nouveau lissage puis on rince soigneusement et on applique la composition oxydante ou fixatrice que l'on laisse agir pendant un temps de 2 à 10 min. environ puis on rince abondamment les cheveux.

On va maintenant donner à titre d'illustration plusieurs exemples de mis en oeuvre de l'invention.

### EXEMPLE 1 :

On prépare selon l'invention, une composition réductrice de déformation permanente des cheveux en procédant de la manière suivante :

On prépare tout d'abord la solution A :
- Monothioglycolate de glycérol 60g
- N-acétylcystéamine 10g
- Glycérire qsp 100g

On prépare ensuite la solution B :
- Lauryléther sulfate d'ammonium 2g
- Parfum qs
- Carbonate acide d'ammonium 3g
- Eau déminéralisée qsp 100g

Après mélange de 30g de la solution A avec 70g de la solution B, le pH de la solution est de 6,9.

On applique cette composition sur des cheveux mouillés, préalablement enroulés sur des bigoudis, et on laisse agir 20 min. à température ambiante. On rince ensuite abondamment à l'eau et on applique alors la composition oxydante suivante :
- Eau oxygénée à 200 volumes 4,8g
- Stabilisant 0,2g
- Alcool oléique à 20 moles d'oxyde d'éthylène 1,5g
- Acide citrique qs pH=3
- Eau déminéralisée qsp 100g

On laisse agir la composition oxydante pendant 10 min., on rince à l'eau puis on enlève les bigoudis.

Après séchage sous casque, on constate que les cheveux présentent de belles boucles avec un bon degré de frisure. Par la suite, au cours du temps les cheveux humidifiés ne présentent pas d'odeur.

En utilisant le même mode opératoire que ci-dessus on a réalisé des ondulations permanentes des cheveux à l'aide des compositions reductrices et oxydantes suivantes :

### EXEMPLE 2 :

### COMPOSITION REDUCTRICE

### SOLUTION A

- Monothioglycolate de glycérol 60g
- Glycérine qsp 100g

### SOLUTION B

- N-acétylcystéamine 6g
- Chlorure d'oléocétyl diméthylammonium 2g
- Carbonate acide d'ammonium 5g
- Parfum qs
- Eau déminéralisée qsp 100g

Après mélange de 30g de la solution A avec 70g de la solution B, le pH avec de la solution est de 7,1.

### COMPOSITION OXYDANTE

- Eau oxygénée à 200 volumes 4,8g
- Stabilisants : sulfate d'hydroxy- 8 quinoléine et phénacétine 0,06g
- Acide citrique qs pH=3
- Eau déminéralisée qsp 100g

On constate que les cheveux présentent les mêmes propriétés que celles décrites ci-dessus à l'exemple 1.

### EXEMPLE 3 :

### COMPOSITION REDUCTRICE

### SOLUTION A

- Monothioglycolate de glycérol 60g
- N-acétylcystéamine 12g
- Glycérine qsp 100g

### SOLUTION B

- Alcool laurique polyoxyéthyléné à 12 moles d'oxyde d'éthyléne 3g
- Copolymère polyvinylpyrrolidone quaternisé ayant un poids moléculaire de 1.000.000 à 20% de matière active vendu sous la dénomination de "GAFQUAT 755" par la Société GAF 1g (m.a)
- Carbonate acide d'ammonium 2,7g
- Parfum qs
- Eau démiralisée qsp 100g

Après mélange de 30g de la solution A avec 70g de la solution B, le pH de la solution est de 6,7.

On applique ce mélange suivant le mode opératoire de l'exemple 1 mais en utilisant une source de chaleur (casque à 40°C) pendant les 20 min..

On utilise la même composition oxydante que celle décrite à l'exemple 2.

### EXEMPLE 4 :

### COMPOSITION REDUCTRICE

Cette composition est identique à celle de l'exemple 1.

### COMPOSITION OXYDANTE

- Bromate de sodium 8g
- Triéthanolamine qs pH=7,5
- Phosphate monosodique, monohydraté 0,3g
- Phosphate trisodique 0,5g
- Eau déminéralisée qsp 100g

On constate que les cheveux présentent les mêmes propriétés que celles décrites à l'exemple 1.

### EXEMPLE 5 :

### COMPOSITION REDUCTRICE

### SOLUTION A

- Monothioglycolate de glycérol 20g
- N-acétylcystéamine 25g
- Glycérine qsp 100g

### SOLUTION B

- Lauryl éther sulfate d'ammonium 3g
- Cabonate acide d'ammonium 1,8g
- Parfum qs
- Eau déminéralisée sqp 100g

Après mélange de 30g de la solution A avec 70g de la solution B, le pH de la solution est de 6,8.

### COMPOSITION OXYDANTE

On utilise la même composition oxydante que celle décrite à l'exemple 2.

On constate que les cheveux présentent les mêmes propriétés que celles décrites à l'exemple 1.

### EXEMPLE 6 :

### COMPOSITION REDUCTRICE

### SOLUTION A

- Monothioglycolate de glycérol 40g
- N-acétylcystéamine 20g
- Glycérine qsp 100g

### SOLUTION B

- Tensio-actif non ionique poly(hydroxypropyléther) préparé par condensation, en catalyse alcaline, de 3,5 moles de glycidol sur un mélange d'alphadiols C₁₁ - C₁₄ selon le procédé décrit dans le Brevet Français n° 2.091.516 3g
- Polymère cationique siliconé vendu par la Société UNION CARBIDE sous la dénomination "UCAR SILICONE ALE 56" à 35% en matière active 1g (m.a)
- Carbonate acide d'ammonium 2g
- Eau déminéralisée qsp 100g

Après mélange de 30g de la solution A avec 70g de la solution B, le pH de la solution est de 6,8.

On applique ce mélange suivant le mode opératoire décrit à l'exemple 1, mais en utilisant une source de chaleur (casque à environ 40°C) pendant les 20 min..

### COMPOSITION OXYDANTE

On utilise la même composition oxydante que celle décrite a l'exemple 2.

On constate que les cheveux presentent les mêmes propriétés que celles décrites à l'exemple 1.

### EXEMPLE 7 :

### COMPOSITION REDUCTRICE

### SOLUTION A

- Monothioglycolate d'éthylène glycol 60 g
- N-acétylcystéamine 10 g
- Glycérine q.s.p 100 g

### SOLUTION B

- Lauryl éther sulfate d'ammonium 2 g
- Carbonate acide d'ammonium 3,5 g
- Parfum qs
- Eau déminéralisée q.s.p 100 g

Après mélange de 30 g de la solution A avec 70 g de la solution B, le pH de la solution est de 6,9.

On applique ce mélange suivant le mode opératoire décrit à l'exemple 1.

### COMPOSITION OXYDANTE

On utilise la même composition oxydante que celle décrite à l'exemple 1.

On constate que les cheveux présentent les mêmes propriétés que celles décrites à l'exemple 1.

## Revendications

1. Composition cosmétique réductrice pour réaliser une déformation permanente des cheveux caractérisée par le fait qu'elle contient en tant qu'agents réducteurs un ester de l'acide thioglycolique et une N-acyl(C₂-C₄)cystéamine, le pH de ladite composition étant inférieur à 8.

2. Composition selon la revendication 1, caractérisée par le fait que l'ester de l'acide thioglycolique est le monothioglycolate de glycérol ou le monothioglycolate d'éthylène glycol.

3. Composition cosmétique selon la revendication 1 caractérisée par le fait que la N-acyl(C₂-C₄)cystéamine est de préférence la N-acétylcystéamine.

4. Composition selon l'une quelconque des revendications 1 à 3 caractérisée par le fait que l'ester de l'acide thioglycolique est présent à une concentration comprise entre 5 et 30% en poids par rapport au poids total de la composition.

5. Composition selon l'une quelconque des revendications 1 à 3 caractérisée par le fait que la N-acyl(C₂-C₄)cystéamine est présente à une concentration comprise entre 0,25 et 30% en poids par rapport au poids total de la composition.

6. Composition cosmétique selon l'une quelconque des revendications précédentes caractérisée par le fait que le rapport en poids entre la N-acyl(C₂-C₄)cystéamine et l'ester de l'acide thioglycolique est compris entre 0,05 et 2.

7. Composition selon l'une quelconque des revendications précédentes caractérisée par le fait qu'elle contient en outre au moins un agent tensio-actif du type non ionique, anionique, cationique ou amphotère en une proportion maximale de 30% mais de préférence comprise entre 0,5 et 10% en poids par rapport au poids total de la composition réductrice.

8. Composition selon l'une quelconque des revendications précédentes caractérisée par le fait qu'elle contient en outre au moins un agent traitant de nature cationique, anionique, non ionique ou amphotère.

9. Composition selon l'une quelconque des revendications précédentes caractérisée par le fait que le pH est de préférence compris entre 5 et 7,5.

10. Composition selon l'une quelconque des revendications précédentes caractérisée par le fait qu'elle contient un solvant, choisi parmi l'éthanol, le propanol, l'isopropanol ou le glycérol, à une concentration maximale de 20% en poids par rapport au poids total de la composition.

11. Conditionnement en plusieurs parties pour la réalisation d'une composition réductrice selon l'une quelconque des revendications 1 à 10 caractérisé par le fait qu'il est constitué d'au moins deux parties, la première partie contenant l'ester de l'acide thioglycolique en milieu anhydre et la deuxième partie contenant en milieu aqueux la N-acyl(C₂-C₄)cystéamine.

12. Conditionnement selon la revendication 11 caractérisé par le fait qu'il est constitué d'une troisième partie contenant en milieu aqueux un régulateur de pH.

13. Conditionnement en plusieurs parties pour la réalisation d'une composition réductrice selon l'une quelconque des revendications 1 à 10 caractérisé par le fait qu'il est constitué d'au moins deux parties, la première partie contenant l'ester de l'acide thioglycolique et la N-acyl(C₂-C₄)cystéamine en milieu anhydre et la deuxième partie contenant le milieu aqueux.

14. Conditionnement selon l'une des revendications 11 et 13 caractérisé par le fait que la partie contenant le milieu aqueux contient un régulateur de pH.

15. Conditionnement selon l'une des revendications 11 à 14 caractérisé par le fait qu'au moins l'une des parties contient en outre au moins un adjuvant cosmétique.

16. Procédé de déformation permanente des cheveux consistant dans une première étape à réduire les liaisons disulfures de la kératine par application sur les cheveux d'une composition réductrice, puis dans une seconde étape à reformer lesdites liaisons par application d'une composition oxydante, caractérisé par le fait que l'étape de réduction est réalisée à l'aide d'une composition cosmétique réductrice telle que revendiquée selon l'une quelconque des revendications 1 à 10.

17. Procédé selon la revendication 16 pour l'ondulation des cheveux caractérisé par le fait que la composition réductrice est appliquée sur des cheveux mouillés, enroulés sur des rouleaux ayant de 4 à 20 mm. de diamètre.

18. Procédé selon la revendication 16 pour le décrêpage ou le défrisage des cheveux, caractérisé par le fait qu'après application sur les cheveux de la composition réductrice, ceux-ci sont soumis à une opération de lissage à l'aide d'un peigne.

19. Procédé selon l'une quelconque des revendications 16 à 18 caractérisé par le fait que l'on laisse agir la composition réductrice pendant un temps compris entre 5 et 60 min..

## Patentansprüche

1. Reduzierende kosmetische Zubereitung zum Erzielen einer dauernden Verformung der Haare, dadurch gekennzeichnet, daß sie als Reduktionsmittel sowohl einen Ester der Thiogykolsäure als auch ein N-Acyl-(C₂-C₄)-cysteamin enthält, wobei der pH der Zubereitung unterhalb von 8 liegt.

2. Zubereitung gemäß Anspruch 1, dadurch gekennzeichnet, daß der Ester der Thioglykolsäure das Monothioglykolat von Glycerin oder von Ethylenglykol ist.

3. Kosmetische Zubereitung gemäß Anspruch 1, dadurch gekennzeichnet, daß das N-Acyl-(C₂-C₄)-cysteamin vorzugsweise das N-Acetylcysteamin ist.

4. Zubereitung gemäß einem der Ansprüche 1 bis 3, dadurch gekennzeichnet, daß der Ester der Thioglykolsäure in einer Konzentration zwischen 5 bis 30 Gew.-%, bezogen auf das Gesamtgewicht der Zubereitung, vorliegt.

5. Zubereitung gemäß einem der Ansprüche 1 bis 3, dadurch gekennzeichnet, daß das N-Acyl-(C₂-C₄)-cysteamin in einer Konzentration im Bereich zwischen 0,25 und 30 Gew.-%, bezogen auf das Gesamtgewicht der Zubereitung, vorliegt.

6. Kosmetische Zubereitung gemäß einem der vorstehenden Ansprüche, dadurch gekennzeichnet, daß das Gewichtsverhältnis des N-Acyl-(C₂-C₄)-cysteamins zu der Thioglykolsäure zwischen 0,05 und 2 liegt.

7. Zubereitung gemäß einem der vorstehenden Ansprüche, dadurch gekennzeichnet, daß sie zusätzlich mindestens ein nichtionisches, anionisches, kationisches oder amphoteres Tensid in einem maximalen Anteil von 30 %, vorzugsweise zwischen 0,5 und 10 Gew.-%, bezogen auf das Gesamtgewicht der reduzierenden Zubereitung, enthält.

8. Zubereitung gemäß einem der vorstehenden Ansprüche, dadurch gekennzeichnet, daß sie darüber hinaus mindestens ein Pflegemittel kationischer, anionischer, nichtionischer oder amphoterer Natur enthält.

9. Zubereitung gemäß einem der vorstehenden Ansprüche, dadurch gekennzeichnet, daß ihr pH vorzugsweise zwischen 5 und 7,5 liegt.

10. Zubereitung gemäß einem der vorstehenden Ansprüche, dadurch gekennzeichnet, daß sie ein Lösungsmittel, ausgewählt aus Ethanol, Propanol, Isopropanol oder Glycerin, in einer Konzentration von maximal 20 Gew.-%, bezogen auf das Gesamtgewicht der Zubereitung, enthält.

11. Kombipackung aus mehreren Teilen zum Herstellen einer reduzierenden Zusammmensetzung gemäß einem der Ansprüche 1 bis 10, dadurch gekennzeichnet, daß sie aus mindestens zwei Bestandteilen besteht, wobei der erste Bestandteil den Ester der Thioglykolsäure in einem nichtwäßrigen Milieu und der zweite Teil das N-Acyl-(C₂-C₄)-cysteamin in einem wäßrigen Milieu enthält.

12. Kombipackung gemäß Anspruch 11, dadurch gekennzeichnet, daß sie einen dritten Bestandteil umfaßt, der einen pH-Regulator in einem wäßrigen Milieu enthält.

13. Kombipackung aus mehreren Teilen zum Herstellen einer reduzierenden Zusammmensetzung gemäß einem der Ansprüche 1 bis 10, dadurch gekennzeichnet, daß sie aus mindestens zwei Bestandteilen besteht, wobei der erste Bestandteil den Ester der Thioglykolsäure und das N-Acyl-(C₂-C₄)-cysteamin in einem nichtwäßrigen Milieu und der zweite Teil eine wäßrige Lösung enthält.

14. Kombipackung gemäß einem der Ansprüche 11 bis 13, dadurch gekennzeichnet, daß der die wäßrige Lösung enthaltende Bestandteil einen pH-Regulator enthält.

15. Kombipackung gemäß einem der Ansprüche 11 bis 14, dadurch gekennzeichnet, daß mindestens einer der Bestandteile zusätzlich mindestens einen kosmetischen Hilfsstoff enthält.

16. Verfahren zur dauernden Verformung der Haare, welches in einem ersten Schritte die Reduktion der Disulfidbindungen des Keratins durch Auftragen einer reduzierenden Zubereitung auf die Haare, und anschließend in einem zweiten Schritt die Wiederherstellung dieser Bindungen durch Auftragen einer oxidierenden Zubereitung umfaßt, wobei das Verfahren dadurch gekennzeichnet ist, daß der Reduktionsschritt mit Hilfe einer reduzierenden kosmetischen Zubereitung, wie sie gemäß einem der Ansprüche 1 bis 10 beansprucht wird, realisiert wird.

17. Verfahren gemäß Anspruch 16 zur Wellung der Haare, dadurch gekennzeichnet, daß die reduzierende Zubereitung auf angefeuchtetes, auf Wickler mit einem Durchmesser von 4 bis 20 mm aufgedrehtes Haar aufgetragen wird.

18. Verfahren gemäß Anspruch 16 zur Glättung oder Entkräuselung von Haaren, dadurch gekennzeichnet, daß auf die Haare eine reduzierende Zubereitung aufgetragen wird, wobei diese einem Verfahren zur Glättung mit Hilfe eines Kammes unterworfen werden.

19. Verfahren gemäß einem der Ansprüche 16 bis 18, dadurch gekennzeichnet, daß man die reduzierende Zubereitung während einer Dauer von 5 bis 60 min einwirken läßt.

## Claims

1. Cosmetic reducing composition for carrying out a permanent-reshaping of hair, characterized in that it contains as reducing agents a thioglycolic acid ester and an N-(C₂-C₄ acyl)cysteamine, the pH of said composition being below 8.

2. Composition according to Claim 1, characterized in that the thioglycolic acid ester is glycerol monothioglycolate or ethylene glycol monothioglycolate.

3. Cosmetic composition according to Claim 1, characterized in that the N-(C₂-C₄ acyl)cysteamine is preferably N-acetylcysteamine.

4. Composition according to any one of Claims 1 to 3, characterized in that the thioglycolic acid ester is present at a concentration of between 5 and 30% by weight relative to the total weight of the composition.

5. Composition according to any one of Claims 1 to 3, characterized in that the N-(C₂-C₄ acyl)cysteamine is present at a concentration of between 0.25 and 30% by weight relative to the total weight of the composition.

6. Cosmetic composition according to any of of the preceding claims, characterized in that the weight ratio of the N-(C₂-C₄ acyl)cysteamine to the thioglycolic acid ester is between 0.05 and 2.

7. Composition according to any one of the preceding claims, characterized in that it contains, in addition, at least one nonionic, anionic, cationic or amphoteric type surfactant in a maximum proportion of 30%, but preferably of between 0.5 and 10%, by weight relative to the total weight of the reducing composition.

8. Composition according to any one of the preceding claims, characterized in that it contains, in addition, at least one treatment agent of a cationic, anionic, nonionic or amphoteric nature.

9. Composition according to any one of the preceding claims, characterized in that the pH is preferably between 5 and 7.5.

10. Composition according to any one of the preceding claims, characterized in that it contains a solvent chosen from ethanol, propanol, isopropanol or glycerol, at a maximum concentration of 20% by weight relative to the total weight of the composition.

11. Multi-part packaging for the production of a reducing composition according to any one of Claims 1 to 10, characterized in that it consists of at least two parts, the first part containing the thioglycolic acid ester in an anhydrous medium and the second part containing the N-(C₂-C₄ acyl) cysteamine in an aqueous medium.

12. Packaging according to Claim 11, characterized in that it consists of a third part containing a pH regulator in an aqueous medium.

13. Multi-part packaging for the production of a reducing composition according to any one of Claims 1 to 10, characterized in that it consists of at least two parts, the first part containing the thioglycolic acid ester and the N-(C₂-C₄ acyl)cysteamine in an anhydrous medium and the second part containing the aqueous medium.

14. Packaging according to one of Claims 11 and 13, characterized in that the part containing the aqueous medium contains a pH regulator.

15. Packaging according to one of Claims 11 to 14, characterized in that at least one of the parts contains, in addition, at least one cosmetic adjuvant.

16. Process for the permanent-reshaping of hair, consisting, in a first step, in reducing the disulphide bonds of the keratin by applying a reducing composition to the hair, and then, in a second step, in re-forming the said bonds by applying an oxidizing composition, characterized in that the reduction step is carried out using a cosmetic reducing composition as claimed according to any one of Claims 1 to 10.

17. Process according to Claim 16 for the waving of hair, characterized in that the reducing composition is applied to wet hair wound on rollers 4 to 20 mm in diameter.

18. Process according to Claim 16 for the straightening or uncurling of hair, characterized in that, after application of the reducing composition to the hair, the latter is subjected to a smoothing operation using a comb.

19. Process according to any one of Claims 16 to 18, characterized in that the reducing composition is left to act for a time between 5 and 60 min.
